# DEMANDE DE BREVET EUROPEEN

(11) **EP 1 231 760 A1**
(43) Date de publication de la demande: **14.08.2002**
(21) Numéro de dépôt: 02290190.4
(22) Date de dépôt: 29.01.2002
(51) Int. Cl.: H04M 1/247, A61F 4/00

(54) **Téléphone de table à commande buccale**

(30) Priorité: 07.02.2001 FR 0101674
(71) Demandeur: Terny, Michel, 86580 Vouneuil sous Biard (FR)
(72) Inventeur: Terny, Michel, 86580 Vouneuil sous Biard (FR)

(57) **Abrégé**

L'invention concerne un appareil téléphonique de table sans combiné dont toutes les fonctions sont mises en oeuvre à l'aide d'un bâtonnet que l'opérateur tient dans sa bouche
Le dispositif est constitué d'un boîtier (1) fixé à un socle lourd (3) posé sur une table dont la face avant (2) peut être positionnée avec un angle de 40 à 70 degrés par rapport à l'horizontale. La prise de ligne s'effectue avec un interrupteur à bascule (5) dont le levier est suffisamment long pour être actionné avec la bouche La numérotation est exécutée avec le bâtonnet de commande (6) en attente dans le doigt de gant (7) que l'opérateur prend dans sa bouche pour percuter chaque touche sans aléa grâce à la grille de guidage (9) placée à quelques millimètres au-dessus du clavier. La conversation bilatérale, en mode duplex, utilise le microphone et le haut-parleur intégrés dans le boîtier.
Le dispositif selon l'invention est particulièrement destine à des personnes handicapées par une paralysie des deux bras.

## Description

L'invention concerne un appareil téléphonique dont toutes les fonctions habituelles peuvent être utilisées à l'aide d'un bâtonnet de commande que l'opérateur tient dans sa bouche.

Actuellement, une personne handicapée par une paralysie des deux bras a besoin de quelqu'un pour décrocher le traditionnel combiné, pour composer le numéro dans le cas d'un appel et ensuite la personne aidante doit tenir l'écouteur contre l'oreille de la personne handicapée et pour terminer, elle doit mettre fin à la communication en reposant le combiné qui vient d 'être utilisé.

Le dispositif objet de l'invention permet de rendre totalement autonomes les personnes handicapées par une paralysie des deux bras, aussi bien pour recevoir des appels téléphoniques que pour procéder, elles-mêmes , à l'appel d'un correspondant puis converser avec celui-ci et rendre la ligne quand elles le souhaitent.

Le dispositif selon l'invention est composé d'un boîtier dont la face avant comporte essentiellement un commutateur de prise de ligne, un clavier téléphonique, un microphone et un haut-parleur ; le dit boîtier est solidaire d'un socle lourd, de 2 à 3 kilogrammes, destiné à être posé sur une table. Le boîtier et sa face avant peuvent être immobilisés avec une inclinaison de 40 à 70 degrés par rapport à l'horizontale. A 3 mm au-dessus des touches du clavier et parallèlement à celui-ci est placée une plaque perforée offrant à l'opérateur un trou de guidage pour le bâtonnet à l'aplomb de chacune des touches. L'équipement de la face avant est complété par un voyant lumineux qui s'illumine quand la ligne est prise et par un doigt de gant qui contient le bâtonnet buccal d'environ 15 centimètres de longueur pour un diamètre d'environ 7 millimètres en attente d'être utilisé pour la numérotation.

Le boîtier contient une carte électronique pour gérer et contrôler toutes les fonctions habituelles d'un appareil téléphonique classique à savoir ::prise de ligne et raccrochage, numérotation pour appeler un correspondant, transmission bilatérale en mode duplex de la conversation établie entre les deux abonnés .

Dans une forme de réalisation préférentielle représentée par les figures (1) et (2) le boîtier rectangulaire (1) peut être basculé d'avant en arrière en pivotant autour de l'axe (4) dont une extrémité est munie d'un écrou de serrage pour immobiliser la face avant (2) avec l'inclinaison désirée. Le socle (3) est rempli de ciment ou de fonte pour l'alourdir enfin d'empêcher son basculement vers l'arrière et son glissement sur le plan de pose pendant la numérotation avec le bâtonnet. En haut, du coté gauche de la face avant est fixé l'interrupteur à bascule de prise de ligne dont le levier de manoeuvre (5) mesure environ 8 centimètres de longueur. Juste en dessous on remarque le voyant indicateur de prise de ligne (8).En haut et à droite le doigt de gant (7) contient le bâtonnet de commande (6) en position favorable pour sa préhension par la bouche de l'opérateur. A 3 mm au-dessus des touches du clavier (11) est positionnée une plaque de 3 millimètres d'épaisseur perforée de 12 trous de 8 millimètres environ de diamètre avec chaque trou situé à l'aplomb de chaque touche. Les figures (3) et (4) montre que l'on réalise ainsi une grille de guidage (9) qui empêche le bâtonnet de glisser au moment de la frappe d'une touche. En haut de la face avant, au centre, se trouve le microphone (10) tandis qu'en partie basse on trouve une zone (12) perforée de multiples trous de 3 millimètres derrière laquelle est fixé le haut-parleur. L'intérieur du boîtier contient une carte électronique équipée de composants à faibles consommations permettant d'alimenter les circuits avec l'énergie disponible sur la ligne téléphonique dès que la connexion est réalisée en agissant sur la prise de ligne (5). De ce fait un unique câble (13) sort de l'arrière du boîtier avec à son extrémité la fiche (14) prévue pour être enfoncée dans la prise murale téléphonique (15), la forme de la fiche et celle de la prise étant adaptée à la réglementation du pays concerné.

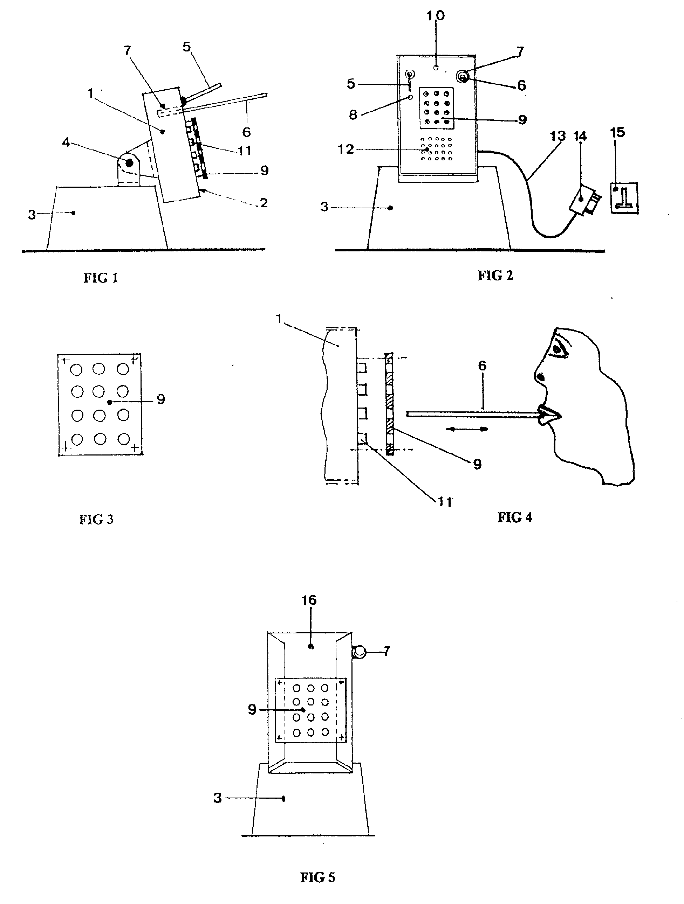

Dans une variante de réalisation la grille de guidage (9) et le doigt de gant (7) qui contient le bâtonnet de commande sont fixés sur un support indépendant du boîtier proprement dit, ce support étant assujetti au socle (3) comme précédemment décrit. Ce support en forme de berceau représenté par la figure (5) permet de recevoir des appareils téléphoniques amovibles et de forme différentes sans avoir à les modifier pour les doter de la grille de guidage (9) et du porte bâtonnet (7), l'un des trous de guidage étant situé à l'aplomb de la touche de prise de ligne.

## Revendications

1. Dispositif permettant à une personne ayant les deux bras paralysés de téléphoner **caractérisé par** le regroupement dans un seul boîtier (1) d'un interrupteur de prise de ligne (5) ,d'un microphone (10), d'un haut-parleur (12) et d'un clavier téléphonique (11) avec une grille de guidage (9) fixée à 3 mm au-dessus des touches, le dit boîtier étant fixé sur un socle (3) alourdi par du ciment ou de la fonte pour empêcher le basculement ou le glissement du boîtier vers l'arrière, pendant la numérotation avec un bâtonnet tenu dans la bouche, l'ensemble étant posé sur une table. devant laquelle vient se positionner l'utilisateur.

2. Dispositif selon la revendication (1) **caractérisé en ce que** l'inclinaison du boîtier (1) peut être réglée entre 40 et 70 degrés par rapport à l'horizontale, sans discontinuité, grâce à un axe de rotation (4) dont une extrémité est munie d'un écrou de serrage pour immobiliser la face avant (2) dans la position souhaitée par l'opérateur.

3. Dispositif selon la revendication (1) **caractérisé par** la présence à quelques mm au dessus du clavier d'une grille de guidage (9) dont chaque trou d'environ 8 millimètres de diamètre est situé à l'aplomb de chaque touche du clavier pour guider l'extrémité du bâtonnet (6) que l'opérateur tient dans sa bouche pour frapper une touche

4. Dispositif selon la revendication (1) **caractérisé par** l'interrupteur de prise de ligne à bascule dont le levier de manoeuvre (5) mesure environ 8 centimètres de longueur pour que l'opérateur puisse le basculer de bas en haut - et réciproquement - avec sa bouche.

5. Dispositif **caractérisé par** la présence sur la face avant (2) du boîtier d'un doigt de gant (7) qui contient le bâtonnet de commande (6) en attente dans une position idéale pour que l'opérateur puisse le saisir facilement avec sa bouche.

6. Dispositif selon les revendications 1,2 3,5 **caractérisé en ce que** la grille de guidage (9) et le doigt de gant (7) peuvent être fixés sur un support (16) indépendant du boîtier électronique proprement dit, ce support étant fixé au socle (3) comme précédemment décrit.

7. Dispositif selon les revendication (3) et (6) **caractérisé par** la possibilité de glisser un appareil téléphonique dans le support (16) de manière à ce que chaque trou de guidage de la grille (9) se trouve à l'aplomb de chaque touche du clavier disposé en dessous.
